# DEMANDE DE BREVET EUROPEEN

(11) **EP 2 332 521 A1**
(43) Date de publication de la demande: **15.06.2011**
(21) Numéro de dépôt: 10194035.1
(22) Date de dépôt: 07.12.2010
(51) Int. Cl.: A61K 8/99, A61Q 19/08

(54) **Microorganismes probiotiques à titre d'actif contre les altérations du microrelief de la peau**

(30) Priorité: 08.12.2009 FR 0958745
(71) Demandeur: L'Oréal, 75008 Paris (FR); Nestec S.A., 1800 Vevey (CH)
(72) Inventeur: Gueniche, Audrey, 92500, Rueil Malmaison (FR); Castiel, Isabelle, 06200, Nice (FR)
(74) Mandataire: Le Coupanec, Pascale A.M.P.

(57) **Abrégé**

L'invention concerne l'utilisation d'au moins un microorganisme probiotique à titre d'actif pour prévenir et/ou traiter des altérations du microrelief de la peau.

## Description

La présente invention concerne l'utilisation de microorganismes probiotiques pour lisser la peau, notamment la peau du visage.

Plus particulièrement, l'invention concerne l'utilisation d'un microorganisme probiotique, notamment du genre *Lactobacillus sp.,* à titre d'actif utile pour traiter et/ou prévenir une altération du microrelief. Egalement, l'invention a pour un objet un procédé pour lisser le microrelief de la peau.

La surface de la peau humaine n'est pas lisse. Elle présente un relief se traduisant par des lignes fines, distinctes des rides, observables à la loupe chez l'enfant et à l'oeil nu chez le vieillard. Ces lignes fines ou sillons s'entrecroisent de sorte à former des structures de formes polygonales, à savoir le microrelief de la peau.

Le nombre et la profondeur des sillons constituant le microrelief de la peau peuvent être affectés par de nombreux facteurs extérieurs ou internes. Parmi les facteurs extrinsèques, on peut citer l'exposition au soleil, l'exposition aux variations de températures et d'humidité, ou l'exposition aux polluants. Parmi les facteurs intrinsèques affectant le microrelief de la peau, on peut citer le stress, la fatigue, des changements hormonaux, une déshydratation de l'épiderme ou une altération de la fonction barrière de la peau, voire le vieillissement. Ces facteurs extrinsèques et intrinsèques tendent à augmenter le nombre et la profondeur des sillons du microrelief.

Ces derniers deviennent plus marqués et visibles à l'oeil nu.

Il s'avère donc important d'être en mesure de prévenir, voire de réduire les altérations du microrelief.

Par ailleurs, les facteurs externes ou internes susceptibles d'altérer le microrelief de la peau peuvent également affecter son teint. En conséquence, des altérations du microrelief et de l'éclat du teint de la peau peuvent se développer fréquemment conjointement.

WO 2007/112996 décrit une composition pour lisser le microrelief de la peau, comprenant différents actifs extraits de végétaux, tels que la génistéine, le resvératrol, ou le bêta-carotène, formulés dans un produit lacté fermenté tel que le yaourt Nestlé LC1.

WO 2006/104730 décrit un complément alimentaire comprenant un microorganisme probiotique pour améliorer l'apparence de la peau, et prévenir les signes cutanés notamment liés à l'âge, comme l'apparition des rides ou un aspect rugueux.

US 2008/206211 propose l'utilisation d'un milieu de culture conditionné par des bactéries pour traiter la déshydratation ou le vieillissement de la peau.

Toutefois, il apparaît qu'aucune des solutions proposées dans l'art antérieur ne s'avèrent satisfaisantes pour prévenir et/ou traiter les altérations du microrelief de la peau.

Ainsi, il existe encore un besoin de disposer de nouveaux actifs utiles pour traiter et/ou prévenir une altération du microrelief de la peau, et en particulier de la peau du visage.

Il existe encore un besoin de disposer de nouveaux actifs utiles pour réduire le nombre et/ou la profondeur des sillons du microrelief de la peau.

Il existe encore un besoin de disposer de nouveaux actifs utiles pour améliorer l'homogénéité de l'orientation des lignes fines du microrelief et d'accroître la densité en corner/cm², caractéristique des peaux jeunes.

Il existe encore un besoin de disposer de nouveaux actifs permettant de conférer un teint lumineux à la peau.

Il existe encore un besoin de disposer de nouveaux actifs utiles pour prévenir et/ou traiter des imperfections de la peau.

La présente invention a pour objet de satisfaire ces besoins.

Selon un premier objet, la présente invention concerne une utilisation cosmétique d'au moins un microorganisme probiotique, notamment du genre *Lactobacillus sρ.,* à titre d'actif utile pour traiter et/ou prévenir une altération du microrelief de la peau.

De manière surprenante, les inventeurs ont observé que l'administration d'une quantité efficace, notamment par voie orale, d'un microorganisme probiotique, notamment du genre *Lactobacillus sp.,* en particulier *Lactobacillus paracasei,* et plus particulièrement *Lactobacillus paracasei* ST11, permettait de réduire significativement le nombre et la profondeur des sillons du microrelief, et d'accroître la densité en corner/cm2.

Selon un mode de réalisation, les altérations du microrelief de la peau considérées par la présente invention ne sont pas liées à l'âge, et en particulier ne sont pas des signes du vieillissement cutané.

L'utilisation de microorganismes probiotiques selon l'invention permet avantageusement de renforcer les propriétés et fonctions barrières de la peau, favorisant ainsi l'intégrité de la peau, et la préservation de son microrelief.

Selon un autre avantage, une utilisation selon l'invention, permet d'optimiser l'assimilation des nutriments apportés par l'alimentation au niveau de la muqueuse intestinale et de promouvoir l'apport en nutriments indispensable au métabolisme cellulaire. La synthèse des différents éléments fonctionnels et structurels de la peau est ainsi favorisée, permettant le maintien de l'homéostasie cutanée et réduction des altérations du microrelief de la peau, voire un lissage de la peau.

Selon encore un autre avantage, une utilisation conforme à l'invention permet de restaurer et de maintenir l'hydratation et la fonction barrière de la peau, ainsi que des tissus sous-jacents, favorisant ainsi la réduction de la profondeur des sillons du microrelief de la peau et réduisant la visibilité de ce dernier.

Selon un mode de réalisation, un microorganisme probiotique considéré dans l'invention est distinct d'un *Lactobacillus johnsonii* formulé dans une composition comprenant en poids :
- 875,42 g de lait entier 1,7 %,
- 10 g de lait écrémé 1,0 %,
- 30 g de yaourt Nestlé LC1,
- 70 g de sucre,
- 3 g de stabilisant,
- 0,08 de saveur tangerine,
- 0,93 g de teavigo,
- 0,17 g de génistéine TG,
- 0,14 g de resvératrol,
- 0,24 g de β-carotène 7 % CWS,
- 10 g d'huile alimentaire ROPUFA '30' n-3.

Selon un mode de réalisation préféré, un microorganisme probiotique convenant à l'invention pour traiter et/ou prévenir une altération du microrelief de la peau peut être avantageusement un *Lactobacillus paracasei,* et de préférence un *Lactobacillus paracasei* ST11.

Selon un autre mode de réalisation préféré, un microorganisme probiotique convenant à l'invention n'est pas associé avec au moins un actif choisi parmi le lait entier, le lait écrémé, le teavigo, la génistéine, le resvératrol, le β-carotène, l'huile alimentaire ROPUFA '30' n-3, et leurs mélanges.

Selon un autre mode de réalisation, un microorganisme probiotique convenant à l'invention n'est pas associé à un mélange comprenant du lait entier, du lait écrémé, du teavigo, de la génistéine, du resvératrol, du β-carotène et de l'huile alimentaire ROPUFA'30'n-3.

Selon encore un autre mode de réalisation, un microorganisme probiotique convenant à l'invention est distinct d'un *Lactobacillus johnsonii* (ou *acidophilus*).

Selon un mode de réalisation, la présente invention a également pour objet un procédé cosmétique de prévention et/ou de traitement de l'altération du microrelief de la peau comprenant l'administration d'au moins un microorganisme probiotique, notamment du genre *Lactobacillus sp.,* notamment tel que défini ci-après.

Selon l'invention, on entend désigner par le terme « peau », l'ensemble de l'épiderme d'un individu, en particulier d'un être humain, et plus particulièrement la peau du décolleté, du cou et du visage, et notamment la peau du visage.

Selon encore un mode de réalisation, la présente invention a pour objet de réduire le nombre de sillons du microrelief de la peau, et/ou de réduire la profondeur des sillons du microrelief de la peau.

Selon encore un mode de réalisation, la présente invention a pour objet d'accroître la densité en corners/cm²de la peau.

Un sillon, ou ligne fine, du microrelief de la peau présente une profondeur maximale de 55 µm. Un sillon de la peau présentant une profondeur supérieure à 55 µm peut être qualifié de ridule (55 à 110 µm) ou ride (supérieur à 110 µm).

Ainsi, les structures du microrelief de la peau considérée dans l'invention ont une profondeur ne dépassant pas 55 µm.

Les sillons ou lignes fines s'entrecroisent et forment des structures de formes polygonales, caractéristiques du microrelief de la peau, et sont distincts des rides ou ridules de la peau. Ces structures définissent des corners, dont la présence en nombre élevé est caractéristique des peaux saines, lisses et notamment jeunes.

La présence et la visibilité des sillons ou lignes fines du microrelief de la peau considérés dans la présente invention peut être accentuée par différents facteurs extrinsèques ou intrinsèques, tels que l'exposition à des variations de températures ou d'humidité, l'exposition au soleil ou aux UV, l'exposition au froid, l'exposition aux polluants, l'exposition au stress ou à la fatigue, ou l'exposition à des changements hormonaux.

La présence ou la visibilité des sillons du microrelief de la peau peut également être affectée par le degré d'hydratation de la peau ou des tissus sous-jacents.

La présence d'un microrelief faiblement marqué est un signe de bonne santé de la peau, et notamment d'une bonne hydratation et d'une homéostasie des propriétés et fonctions barrières bien régulées.

L'utilisation d'un microorganisme probiotique conforme à l'invention, et notamment du genre *Lactobacillus sp.,* permet avantageusement de prévenir, de traiter ou renverser les effets délétères de ces facteurs extrinsèques ou intrinsèques sur le microrelief de la peau.

Selon un mode de réalisation, le microrelief considéré dans la présente invention est avantageusement le microrelief de la peau du visage.

Selon un mode de réalisation, un microorganisme probiotique convenant à l'invention peut être administré par voie orale, topique ou parentérale, et en particulier par voie orale.

Une mise en oeuvre d'un microorganisme selon l'invention se fait nécessairement en une quantité efficace, i.e. une quantité permettant au microorganisme probiotique de manifester ses propriétés d'actif au regard des altérations du microrelief de la peau à prévenir et/ou traiter.

Au sens de la présente invention, on entend par « prévenir » le fait de diminuer au moins en partie le risque de manifestation d'un phénomène donné, i.e. dans la présente invention une altération du microrelief de la peau. La diminution partielle implique que le risque demeure mais à un degré moindre qu'avant la mise en oeuvre de l'invention.

### Microorganismes

Au sens de la présente invention, on entend par « microorganisme probiotique », un microorganisme vivant qui, lorsqu'il est consommé en quantité adéquate, a un effet positif sur la santé de son hôte (« Joint FAO/WHO Expert Consultation on Evaluation of Health and Nutritional Properties of Probiotic in Food Including Powder Milk with Live Lactic Acid Bacteria, 6 octobre 2001 »), et qui peut en particulier améliorer l'équilibre microbien intestinal.

Selon un mode de réalisation, un microorganisme probiotique selon l'invention peut être mis en oeuvre sous une forme isolée ou purifié, c'est-à-dire non mélangée à un ou des composé(s) susceptible(s) de lui être associé(s) dans son milieu d'origine.

Selon un autre mode de réalisation, un microorganisme probiotique selon l'invention peut être mis en oeuvre sous une forme vivante, semi-active, inactivée ou morte.

Selon un mode de réalisation avantageux de l'invention, un microorganisme probiotique peut être avantageusement mis en oeuvre sous forme inactivée ou morte.

Un microorganisme probiotique administré par voie topique peut être avantageusement mis en oeuvre sous forme inactivée ou morte.

Un microorganisme probiotique administré par voie orale peut être avantageusement mis en oeuvre sous forme vivante.

Au sens de l'invention, un microorganisme « inactivé » est un microorganisme qui n'est plus capable, temporairement ou définitivement, de former des colonies en cultures. Au sens de l'invention, un microorganisme « mort » est un microorganisme qui n'est plus capable, définitivement, de former des colonies en cultures. Les microorganismes morts ou inactivés peuvent avoir les membranes cellulaires intactes ou rompues. Ainsi le terme « inactivé » désigne également les extraits et lysats de microorganismes comme détaillés ci-après. L'obtention de microorganismes morts ou inactivés peut être effectuée par toute méthode connue de l'homme de l'art.

Selon un mode de réalisation avantageux, les microorganismes probiotiques mis en oeuvre selon l'invention sont au moins en partie inactivés ou morts.

Par microorganismes probiotiques au moins en partie inactivés ou morts, on désigne une préparation de microorganismes probiotiques conformes à l'invention comprenant au moins 80 %, en particulier au moins 85 %, plus particulièrement au moins 90 %, voire au moins 95 %, ou au moins 99 % de microorganismes probiotiques inactivés ou morts exprimés par rapport à la totalité des microorganismes probiotiques vivants non inactivés contenus dans la préparation initiale avant d'être soumise à un procédé d'inactivation ou pour tuer les microorganismes.

Le taux d'inactivation ou de mortalité obtenu dépend des conditions d'application de la méthode utilisée qui sont ajustées par l'homme de l'art selon le taux d'inactivation ou de mortalité à obtenir.

Selon un mode de réalisation, l'invention comprend la mise en oeuvre d'une préparation comprenant 100 % de microorganismes probiotiques inactivés ou morts.

Un microorganisme probiotique inactivé convenant à l'invention peut être préparé par irradiation, inactivation thermique ou lyophilisation d'une préparation de microorganisme. Ces méthodes sont connues de l'homme de l'art.

Plus particulièrement, l'inactivation de microorganismes probiotiques par irradiation peut comprendre la mise en oeuvre de rayons gamma, de rayons X, une exposition aux UV, de chaleur, ou d'une dépression. Le type de traitement, l'intensité, la dose et le temps d'exposition sont ajustés par l'homme de l'art selon la quantité et la nature des microorganismes probiotiques à inactiver.

Selon une variante préférée de réalisation, un microorganisme probiotique convenant à l'invention est mis en oeuvre sous une forme inactivée obtenue par irradiation, notamment irradiation gamma.

L'inactivation par lyophilisation peut être effectuée par toute méthode connue dans le domaine. Avantageusement, des microorganismes probiotiques inactivés par lyophilisation peuvent être remis en culture.

L'inactivation thermique peut être réalisée en incubant les microorganismes probiotiques de l'invention pendant une période de temps prolongée, par exemple au moins deux heures, à 170 C. L'inactivation thermique peut également être opérée par autoclavage en soumettant les microorganismes probiotiques de l'invention à une température de 121 °C, pendant au moins 20 minutes et à une pression atmosphérique de 2 bars.

Alternativement, l'inactivation thermique peut être effectuée en soumettant les microorganismes probiotiques à une température de congélation, pendant une période de temps prolongée.

Un microorganisme probiotique selon l'invention peut être mis en oeuvre sous forme entière, c'est-à-dire pour l'essentiel dans sa forme native, ou sous forme d'extraits ou de lysats de suspensions désintégrées comprenant des fractions et/ou des métabolites de ce microorganisme.

Au sens de l'invention, le terme « métabolite » désigne toute substance issue du métabolisme des microorganismes, et notamment sécrétée par les microorganismes considérés selon l'invention et dotée également d'une efficacité pour le traitement et/ou la prévention des altérations du microrelief de la peau.

Au sens de l'invention, le terme « fraction » désigne plus particulièrement un fragment dudit microorganisme et doté d'une efficacité pour le traitement et/ou la prévention des altérations du microrelief de la peau par analogie audit microorganisme entier.

Un extrait ou lysat convenant à l'invention peut être préparé à partir des microorganismes probiotiques en fin de phase de croissance.

Selon un mode préféré de réalisation, un microorganisme probiotique convenant à l'invention peut être mis en oeuvre sous forme d'un lysat.

Un lysat au sens de l'invention désigne communément un matériau obtenu à l'issue de la destruction ou dissolution de cellules biologiques par un phénomène dit de lyse cellulaire provoquant ainsi la libération des constituants biologiques intracellulaires naturellement contenus dans les cellules du microorganisme considéré.

Au sens de la présente invention, le terme lysat est utilisé indifféremment pour désigner l'intégralité du lysat obtenu par lyse du microorganisme concerné ou seulement une fraction de celui-ci.

Le lysat mis en oeuvre est donc formé en tout ou partie des constituants biologiques intracellulaires et des constituants des parois et membranes cellulaires.

Avantageusement, un lysat utilisé pour l'invention peut être l'intégralité du lysat obtenu par lyse du microorganisme concerné.

Cette lyse cellulaire peut être accomplie par différentes technologies, telles que par exemple un choc osmotique, un choc thermique, par ultrasons, ou encore sous contrainte mécanique de type centrifugation.

Plus particulièrement, ce lysat peut être obtenu selon la technologie décrite dans le brevet US 4,464,362, et notamment selon le protocole suivant.

En particulier, un lysat de l'invention peut être obtenu par désintégration aux ultra-sons d'un milieu comprenant des microorganismes probiotiques en suspension afin d'en libérer les fractions cytoplasmiques, les fragments de paroi cellulaire et les produits issus du métabolisme. Puis tous les composants dans leur distribution naturelle sont ensuite stabilisés dans une solution aqueuse faiblement acide.

Un microorganisme, un extrait ou un lysat peuvent être mis en oeuvre sous différentes formes, tel qu'une solution, un surnageant de culture, une poudre, éventuellement lyophilisée, ou un concentré.

Selon un mode de réalisation, un microorganisme probiotique convenant à l'invention peut être choisi parmi des *Lactobacillus sp.,* des *Bifidobacterium sp.,* des *Cocci,* des levures, des bactéries sporulées, et leurs mélanges.

Selon un mode de réalisation, un microorganisme convenant à l'invention est préférentiellement choisi parmi :
- des ascomycètes : notamment *Saccharomyces,* notamment *S. cerevisiae* ou *S. boulardii, Yarrowia,* notamment *Y. Lipolitira, Kluyveromyces, Torulaspora, Schizosaccharomyces pombe, Debaromyces, Candida, Pichia, Aspergillus,* notamment *K. lactis* et *Penicillium,*
- des bactéries sporulées : notamment *Bacillus sp.,* notamment *B. cereus* var *toyo* ou *B. subtilis, B. coagulans, B. licheniformis, Escherichia sp.,* notamment *E. coli strain nissle, Propionibacterium sp.,* notamment *P. freudenreichii, Bacteroides sp., Fusobacterium sp.,* et *Weissella sp.,*
- des *Cocci :* notamment *Melissococcus sp., Staphylococcus sp.,* notamment *S. carnosus* ou *S. xylosus, Peptostrepococcus sp., Pediococcus sp.,* notamment *P. acidilactici, Micrococcus sp., Leuconostoc sp.,* notamment *L. mesenteroides* subsp *dextranicum, Aerococcus sp., Oenococcus sp., Enterococcus sp.,* notamment *E. faecalis* ou *E. faecium, Lactococcus sp.,* notamment *L. lactis subsp lactis* ou *L. cremoris, Sporolactobacillus sp.,* notamment *S. inulinus,* et *Streptococcus sp.,* notamment *S. salivarius* subsp. *thermophilus* ou *S. thermophilus,*
- des *Lactobacillus species :* notamment *L. paracasei, L. acidophilus, L. amylovorus, L. casei, L. rhamnosus, L. brevis, L. crispatus, L. delbrueckii* subsp. *lactis, L. bulgaricus, L. fermentum, L. helveticus, L. gallinarum, L. gasseri, L. johnsonii, L. plantarum, L. reuteri, L. salivarius, L. alimentarius, L. curvatus, L. casei* subsp. *casei, L. sake,* et
- des bifidobactéries ou *Bifidobacterium species : B. adolescentis, B. animalis, B. bifidum, B. breve, B. lactis, B. longum, B. infantis, B. pseudocatenulatum,*
- et leurs mélanges.

Plus particulièrement, il peut s'agir d'un microorganisme probiotique choisi parmi *Lactobacillus sp., Sporolactobacillus sp., Enterococcus sp., Lactococcus sp., Bacillus sp., Streptococcus sp., Pediococcus sp., Leuconostoc sp.* ou *Bififobacterium sp.,* et en particulier choisi parmi *Lactobacillus sp., Bifidobacterium sp.,* et leurs mélanges.

Comme exemple de microorganismes probiotiques convenant à l'invention, on peut citer *Bifidobacterium adolescentis, B. animalis, B. bifidum, B. breve, B. lactis, B. longum, B. infantis, B. pseudocatenulatum, Lactobacillus acidophilus* NCFB 1748, *L. paracasei, L. amylovorus, L. casei* (*Shirota*), *L. rhamnosus* souche GG, *L. brevis, L. crispatus, L. bulgaricus, L. delbrueckii* subsp. *lactis, L. fermentum, L. helveticus, L. gallinarum, L. gasseri, L. johnsonii* CNCM I-1225, *L. plantarum, L. reuteri, L. salivarius, L. alimentarius, L. curvatus, L. casei* subsp. *casei, L. sake, Enterococcus faecalis, E. faecium, Lactococcus lactis, L. lactis* subsp *lactis, L. lactis* subsp *cremoris, Leuconostoc mesenteroides* subsp *dextranicum, Pediococcus acidilactici, Sporolactobacillus inulinus, Streptococcus salvarius* subsp. *thermophilus, S. thermophilus, Staphylococccus carnosus, S. xylosus, Saccharomyces cerevisiae, S. boulardii, Bacillus cereus* var *toyo, B. cereus* var *subtilis, B. coagulans, B. licheniformis, Escherichia coli strain nissle, Propionibacteriumfreudenreichii,* et leurs mélanges.

A titre d'exemples plus spécifiques de microorganismes probiotiques convenant à l'invention, on peut avantageusement citer *Bifidobacterium bifidum, B. infantis, B. longum, Lactobacillus acidophilus, L. alimentarius, L. casei subsp. casei, L. casei sirota, L. paracasei, L. curvatus, L. delbruckii* subsp. *lactis, L. gasseri, L. johnsonii, L. reuteri, L. rhamnosus* (*Lactobacillus GG), L. sake, Lactococcus lactis, Streptococcus thermophilus, Staphylococccus carnosus, S. xylosus,* et leurs mélanges.

A titre plus particulier de microorganismes probiotiques convenant à l'invention, on peut citer *Lactobacillusjohnsonii* ou *acidophilus, L. reuteri, L. rhamnosus, L. paracasei, L. casei, L. alimentarius, L. curvatus, L. delbruckii* subsp. *lactis, L. gasseri, L. sake, Bifidobacterium bifidum, B. breve, B. longum, B. animalis, B. lactis, B. infantis, B. adolescentis, B. pseudocatenulatum,* et leurs mélanges.

Les espèces convenant tout particulièrement à l'invention sont *Lactobacillus johnsonii, L. paracasei, Bifidobacterium adolescentis, B. longum* respectivement déposés suivant le traité de Budapest avec l'Institut Pasteur (28 rue du Docteur Roux, F-75024 Paris cedex 15) les 30/06/92, 12/01/99, 15/04/99 et le 15/04/99 sous les désignations suivantes CNCM I-1225, CNCM I-2116, CNCM I-2168 et CNCM I-2170, et le genre *Bifidobacterium lactis (Bb 12)* (ATCC27536) ou *Bifidobacterium longum* (BB536). La souche de *Bifidobacterium lactis* (ATCC27536) peut être obtenue chez Hansen (Chr. Hansen A/S, 10-12 Boege Alle, P.O. Box 407, DK-2970 Hoersholm, Danemark).

Avantageusement, un microorganisme convenant à l'invention peut être un microorganisme probiotique à acide lactique, qui produit de l'acide lactique par fermentation du sucre.

Selon un mode de réalisation préféré, un microorganisme probiotique convenant à l'invention, par exemple par voie topique ou orale, et notamment par voie orale, peut en particulier être un microorganisme du genre *Lactobacillus sp..*

De manière préférée, un microorganisme du genre *Lactobacillus sp.* convenant à l'invention peut être choisi parmi *Lactobacillus johnsonii, L. acidophilus, L. reuteri, L. paracasei, L. casei,* et leurs mélanges.

Selon un mode de réalisation préféré, un microorganisme convenant à l'invention peut être choisi parmi *Lactobacillus paracasei, L. johnsonii, L. acidophilus,* et leurs mélanges.

Selon un mode de réalisation préféré, un microorganisme convenant à l'invention peut être un *Lactobacillus paracasei.*

Selon un autre mode de réalisation préféré, un microorganisme convenant à l'invention peut en particulier être la souche *Lactobacillus paracasei* ST11 déposée suivant le traité de Budapest auprès de l'Institut Pasteur (28 rue du Docteur Roux, F-75024 Paris cedex 15) le 30/06/92 sous la désignation CNCM I-2116.

Selon un mode de réalisation, un microorganisme probiotique convenant à l'invention est mis en oeuvre par voie orale, parentérale ou par voie topique.

On entend par voie « parentérale », une voie autre que les voies orale et topique. Une voie parentérale convenant à l'invention peut être, par exemple, la voie nasale.

Selon un mode préféré de réalisation, un microorganisme probiotique convenant à l'invention est mis en oeuvre par voie topique.

La voie topique permet avantageusement d'obtenir une action locale à l'égard de l'effet recherché.

Selon un autre mode préféré de réalisation, un microorganisme probiotique convenant à l'invention est mis en oeuvre par voie orale ou par voie parentérale, et en particulier par voie orale.

La voie orale ou la voie parentérale permettent avantageusement d'obtenir une action globale à l'égard de l'effet recherché.

Un microorganisme de l'invention peut être formulé dans une composition à raison d'au moins 0,0001 % exprimé en poids sec, en particulier à raison de 0,0001 à 30 %, en particulier à raison de 0,001 à 20 % et plus particulièrement à raison de 0,01 à 15 % en poids, en particulier de 0,1 à 10 % en poids, et notamment de 1 % à 5 % en poids par rapport au poids total de la composition le contenant.

D'une manière générale, une composition selon l'invention destinée à être administrée par voie orale peut comprendre pour les microorganismes vivants de 10³ à 10¹⁵ ufc/g, en particulier de 10⁵ à 10¹⁵ ufc/g et plus particulièrement de 10⁷ à 10¹² ufc/g de microorganismes vivants par gramme de composition ou à des doses équivalentes calculées pour les microorganismes inactivés ou morts ou pour des fractions de microorganisme ou pour des métabolites produits.

En particulier, dans une composition administrée par voie orale, la concentration microorganisme et/ou fraction et/ou métabolite correspondant peut être ajustée de manière à correspondre à des doses, exprimées en équivalent de microorganisme, variant de 5.10⁵ à 10¹³ ufc/j et en particulier de 10⁸ à 10¹¹ ufc/j.

Une composition à application topique selon l'invention, peut comprendre généralement de 0,0001 % à 30 % en poids de microorganismes par gramme de composition, en particulier à raison de 0,001 à 20 % et plus particulièrement à raison de 0,01 à 15 % en poids, en particulier de 0,1 à 10 % en poids, et notamment de 1 % à 5 % en poids de microorganismes par rapport au poids total de la composition le contenant.

Dans le cas particulier d'une administration par voie topique, il peut être avantageux de mettre en oeuvre des microorganismes sous forme inactivée, voire morte, notamment sous la forme d'un extrait ou d'un lysat.

Un microorganisme peut également être inclus dans une composition sous forme de fractions de composants cellulaires ou sous la forme de métabolites, en particulier sous la forme d'un lysat. Le ou le(s) microorganisme(s), métabolite(s) ou fraction(s) peu(ven)t également être introduit(s) sous la forme d'une poudre lyophilisée, d'un surnageant de culture et/ou le cas échéant sous une forme concentrée.

Lorsqu'une composition comprend des métabolites, les teneurs en métabolites dans les compositions correspondent sensiblement aux teneurs susceptibles d'être produites par 10³ à 10¹⁵ ufc, en particulier 10⁵ à 10¹⁵ ufc, et plus particulièrement 10⁷ à 10¹² ufc de microorganismes vivants par gramme de composition.

L'expression de la quantité de métabolites ou de fractions d'un microorganisme en « ufc », ou de microorganismes morts, vise à désigner la quantité de ce microorganisme utilisée pour la préparation des métabolites ou des fractions de ce microorganisme.

### Compositions

Un microorganisme probiotique convenant à l'invention est avantageusement formulé dans une composition pouvant se présenter sous toutes formes galéniques normalement disponibles pour le mode d'administration retenu.

Une composition selon l'invention comprend un milieu physiologiquement ou pharmaceutiquement acceptable.

Une composition selon l'invention peut être administrée par voie orale, parentérale, notamment sous-cutanée ou intradermique, ou topique.

De manière préférée, une composition de l'invention peut être administrée par voie topique.

Selon un mode de réalisation, une composition par voie topique selon l'invention peut avantageusement être formulée sous toute forme galénique convenant au soin de la peau et des muqueuses et peuvent se présenter sous forme d'onguent, de crèmes, de laits, de pommades, de poudres, de tampons imbibés, de solutions, de gels, de sprays, de lotions, ou de suspensions. Elles peuvent également se présenter sous forme de microsphères ou nanosphères ou de vésicules lipidiques ou polymériques ou de patchs polymériques et d'hydrogels permettant une libération contrôlée. Ces compositions par voie topique peuvent se présenter soit sous forme anhydre, soit sous forme aqueuse selon l'indication dermocosmétique.

Une compositions destinée à une administration par voie topique peut être une solution aqueuse, hydroalcoolique ou huileuse, une solution ou une dispersion du type lotion ou sérum, une émulsion de consistance liquide ou semi-liquide du type lait, obtenue par dispersion d'une phase grasse dans une phase aqueuse (H/E) ou inversement (E/H), une suspension ou une émulsion, de consistance molle, semi-solide ou solide, du type crème ou du type gel aqueux ou anhydre, une émulsion multiple (E/H/E ou H/E/H), une microémulsion, une nano-émulsion, une préparation de microcapsules, une préparation de microparticules, ou une dispersion vésiculaire de type ionique et/ou non ionique, ou une dispersion cire/phase aqueuse.

Selon un mode préféré de réalisation, une composition par voie topique peut se présenter sous la forme d'une solution, d'une crème, d'un gel, d'une émulsion, d'une mousse ou d'une composition pour aérosol contenant un agent propulseur.

Selon un mode préféré de réalisation, une composition par voie topique peut également se présenter sous la forme d'un système transdermique permettant une libération active ou passive du/des actif(s) par transdermie, par exemple de type patch ou gel patch (hydrogel).

Ces compositions sont préparées selon les méthodes usuelles.

Une composition selon l'invention peut constituer une composition de traitement ou de soin de la peau ou du cuir chevelu, ou une composition de protection solaire ou de bronzage artificiel, ou encore un produit nettoyant ou démaquillant de la peau, un produit déodorant ou encore un composé parfumant.

Une telle composition peut être alors non colorée ou faiblement colorée, et peut contenir éventuellement des actifs cosmétiques ou dermatologiques additionnels, notamment comme indiqué ci-après. Elle peut alors être utilisée comme base de soin pour la peau ou les lèvres, par exemple sous forme d'un baume à lèvres, protégeant les lèvres du froid et/ou du soleil et/ou du vent, comme une crème de soin de jour ou de nuit pour la peau du visage et/ou du corps.

Elle peut également se présenter sous forme de shampooing traitant ou non, colorant ou non, ou d'après-shampooing.

Une composition selon l'invention peut également constituer une composition cosmétique colorée et notamment une composition de maquillage de la peau et/ou des muqueuses, en particulier une telle composition peut être un fond de teint, un blush, un fard à joues ou à paupières, un composé anti-cernes en stick, un rouge à lèvres ou un brillant à lèvres, présentant éventuellement des propriétés de soin ou de traitement. De préférence, il pourra s'agir d'une composition de maquillage colorée (beige ou verte) destinée à corriger la couleur du teint.

Une composition administrée par voie topique peut notamment constituer une crème de nettoyage, de protection, de traitement ou de soin, une lotion, une gel ou une mousse pour le soin de la peau, comme une lotion de nettoyage ou de désinfection, une composition pour le bain ou une composition déodorante.

Une composition selon l'invention peut également consister en une préparation solide constituant un savon ou un pain de nettoyage.

Lorsqu'une composition de l'invention est une émulsion, la proportion de la phase grasse peut aller de 5 à 80 % en poids, et de préférence de 10 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique et/ou dermatologique. L'émulsionnant et le co-émulsionnant peuvent être présents, dans la composition, en une proportion allant de 0,3 à 30 % en poids, et de préférence de 0,5 à 20 % en poids par rapport au poids total de la composition.

Lorsque la composition de l'invention est une solution ou un gel huileux, la phase grasse peut représenter plus de 90 % du poids total de la composition.

De façon connue, les formes galéniques dédiées à une administration topique peuvent contenir également des adjuvants habituels dans le domaine cosmétique, pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

Comme matières grasses utilisables dans l'invention, on peut citer les huiles minérales comme par exemple le polyisobutène hydrogéné et l'huile de vaseline, les huiles végétales comme par exemple une fraction liquide du beurre de karité, l'huile de tournesol et d'amandes d'abricot, les huiles animales comme par exemple le perhydrosqualène, les huiles de synthèse notamment l'huile de Purcellin, le myristate d'isopropyle et le palmitate d'éthyl hexyle, les acides gras insaturés et les huiles fluorées comme par exemple les perfluoropolyéthers. On peut aussi utiliser des alcools gras, des acides gras comme par exemple l'acide stéarique et comme par exemple des cires notamment de paraffine, de carnauba et la cire d'abeilles. On peut aussi utiliser des composés siliconés comme les huiles siliconées et par exemple la cyclométhicone et la diméthicone, les cires, les résines et les gommes siliconées.

Comme émulsionnants utilisables dans l'invention, on peut citer par exemple le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO^{®} par la société HENKEL, le mélange de PEG-6/PEG-32/Glycol Stéarate vendu sous la dénomination de Tefose^{®} 63 par société GATTEFOSSE, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (200E).

Comme solvants utilisables dans l'invention, on peut citer les alcools inférieurs, notamment l'éthanol et l'isopropanol, le propylène glycol.

La composition de l'invention peut également contenir de façon avantageuse une eau thermale et/ou minérale, notamment choisie parmi l'eau de Vittel, les eaux du bassin de Vichy et l'eau de la Roche Posay.

Comme gélifiants hydrophiles, on peut citer les polymères carboxyliques tel que le carbomer, les copolymères acryliques tels que les copolymères d'acrylates/alkylacrylates, les polyacrylamides et notamment le mélange de polyacrylamide, C₁₃₋₁₄ Isoparaffine et Laureth-7 vendu sous le nom de Sepigel 305^{®} par la société SEPPIC, les polysaccharides comme les dérivés cellulosiques tels que les hydroxyalkylcelluloses et en particulier les hydroxypropylcellulose et hydroxyéthylcellulose, les gommes naturelles telles que les guar, caroube et xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

Dans le cas d'une composition conforme à l'invention pour une administration par voie orale, on privilégie l'utilisation d'un support ingérable.

Le support ingérable peut être de nature diverse selon le type de composition considérée.

Conviennent ainsi, notamment, comme supports alimentaires des comprimés, des gélules ou des tablettes, des suspensions, des suppléments oraux sous forme sèche et des suppléments oraux sous forme liquide.

Conviennent également comme supports alimentaires, le lait, le yaourt, le fromage, les laits fermentés, les produits fermentés à base de lait, des glaces, des produits à base de céréales ou des produits à base de céréales fermentées, des poudres à base de lait, des formules pour enfants et nourrissons, des produits alimentaires de type confiserie, le chocolat, les céréales, ou des aliments pour animaux en particulier domestiques.

Par composition pour voie orale, on entend par exemple des compositions nutritionnelles, nutraceutiques, cosméceutiques, ou pharmaceutiques, comprenant au moins un microorganisme probiotique selon l'invention.

La formulation des compositions pour voie orale selon l'invention peut être réalisée par tout procédé usuel connu de l'homme de l'art pour produire des solutions buvables, des dragées, des gélules, des gels, des émulsions, des comprimés à avaler ou à croquer, des capsules, notamment des capsules molles ou dures, des granulés à dissoudre, des sirops, des aliments solides ou liquides et des hydrogels permettant une libération contrôlée

En particulier, un microorganisme probiotique selon l'invention peut être incorporé dans toutes formes de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.

Selon un mode de réalisation, une composition selon l'invention administrée par voie orale peut être formulée sous forme de dragées, de gélules, de gels, d'émulsions, de comprimés, de capsules, d'hydrogels, de barres alimentaires, de poudres, compactées ou non, de suspensions ou solutions liquides, de confiseries, de laits fermentés, de fromages fermentés, de chewing-gum, de pâte dentifrice ou de solutions spray.

Les compositions par voie orale peuvent se présenter soit sous forme anhydre, soit sous forme aqueuse selon l'indication dermocosmétique.

Un microorganisme probiotique de l'invention peut être par ailleurs formulé avec les excipients et composants usuels pour de telles compositions orales ou compléments alimentaires, à savoir notamment des composants gras et/ou aqueux, des agents humectants, épaississants, conservateurs, des agents de texture, de saveur et/ou d'enrobage, des agents antioxydants, conservateurs.

Les agents de formulation et excipients pour composition orale, et notamment pour compléments alimentaires, sont connus dans ce domaine et ne font pas ici l'objet d'une description détaillée.

Selon l'indication privilégiée de l'utilisation d'un microorganisme probiotique de l'invention, une composition de l'invention sera une composition cosmétique, dermatologique ou pharmaceutique.

En particulier, la composition selon l'invention peut être une composition alimentaire pour la consommation humaine. Il peut s'agir en particulier d'aliments complets nutritionnels, de boissons, d'eaux minérales, de soupes, de suppléments diététiques et d'aliments de substitution, de barres nutritionnelles, de confiseries, de produits à base de lait fermenté ou non, de yaourts, de poudres à base de lait, de produit de nutrition entérale, de compositions pour enfants et/ou nourrissons, de produits à base de céréales fermentées ou non, de glaces, de chocolat, de café, de produits « culinaires » tels que de la mayonnaise, la purée de tomate, ou des assaisonnements pour salades.

La composition selon l'invention peut également être destinée aux animaux, notamment aux animaux domestiques, tels que les chats et les chiens, et être formulée sous forme d'aliments ou de compléments alimentaires pour animaux.

Selon un mode de réalisation préféré, un microorganisme probiotique convenant à l'invention peut être mis en oeuvre par voie parentérale, en particulier par voie sous-cutanée ou intradermique.

Dans une telle mise en oeuvre, un microorganisme probiotique convenant à l'invention peut être conditionné sous la forme d'une solution isotonique stérile aqueuse ou non aqueuse, sous forme de dispersion, de suspension ou d'émulsion préparée le cas échéant juste avant l'administration, à partir d'une poudre stérile, par exemple lyophilisée, qui est alors reconstituée sous la forme d'une solution stérile injectable ou d'une dispersion au moment de son utilisation.

Par « stérile », on entend qualifier une formulation apte à garantir l'innocuité requise pour une administration intraépidermique et/ou intradermique et/ou sous-cutanée.

Une composition convenant à l'invention peut comprendre tout excipient habituellement utilisé dans le domaine des solutions stériles injectables.

L'administration d'une composition de l'invention par voie parentérale peut être effectuée par toute technique d'injection convenant à une injection intraépidermique et/ou intradermique et/ou sous-cutanée. Ainsi, une telle administration peut être effectuée au moyen d'une aiguille habituellement utilisée pour réaliser une injection intraépidermique et/ou intradermique et/ou sous-cutanée, convenant pour la mésothérapie.

### Actif additionnel

Un microorganisme probiotique selon l'invention peut avantageusement être mis en oeuvre en association avec un actif additionnel, notamment cosmétique ou pharmaceutique.

Avantageusement, un tel actif additionnel, cosmétique ou pharmaceutique, peut être destiné à exercer un effet cosmétique, de soin ou d'hygiène sur la peau, les cheveux, les cils, les poils et/ou le cuir chevelu, et préférentiellement sur la peau.

Les actifs additionnels sont choisis par l'homme du métier de sorte à ce qu'ils ne nuisent pas à l'effet des microorganismes probiotiques de l'invention.

En particulier, un actif additionnel convenant à l'invention pourra être choisi parmi les actifs destinés à renforcer la barrière cutanée.

Selon un autre mode de réalisation, on peut associer à un microorganisme selon l'invention des agents actifs destinés à la prévention et/ou au traitement des affections cutanées.

A titre d'actif additionnel utilisable, on peut citer :
- les vitamines, telles que les vitamines A, B5, B6, B8, C, D, E, ou PP (vitamine B3 ou niacine),
- les anti-oxydants, tels que les curcuminoïdes ; les caroténoïdes, notamment un caroténoïde choisi parmi le β-carotène, le lycopène, l'astaxanthine, la zéaxanthine et la lutéine; des composés polyphénols, les flavonoïdes tels que les catéchines; les proanthocyanidines, les anthocyanines, les OPC (oligomères procyanidoliques) ; les ubiquinones; les extraits de café contenant des polyphénols et/ou des diterpènes; les extraits de chicorés; les extraits de ginkgo biloba; les extraits de raisins riches en proanthocyanidines ; les extraits de piment; les extraits de soja,
- les minéraux, tels que le zinc, le calcium, le magnésium, le cuivre, le fer, l'iode, le manganèse, le sélénium, le chrome (III),
- les sucres,
- les acides aminés, notamment les acides aminés soufrés, tels que des précurseurs de glutathion, la taurine, les acides aminés du sélénium.
- les acides gras polyinsaturés 3 et 6,
- des prébiotiques, notamment choisis parmi les oligosaccharides, produits à partir du glucose, galactose, xylose, maltose, sucrose, lactose, amidon, xylane, l'hémicellulose, l'inuline, les gommes de type acacia par exemple, ou un de leurs mélanges. Plus particulièrement, l'oligosaccharide comprend au moins un fructo-oligosaccharide. Plus particulièrement, ce prébiotique peut comprendre un mélange de fructo-oligosaccharide et d'inuline,
- les phytostérols, comme le resvératrol,
- l'hespéridine,
- et leurs mélanges.

Selon un mode de réalisation, dans les formes galéniques topiques, on peut utiliser plus particulièrement comme actifs additionnels hydrophiles des protéines ou des hydrolysats de protéine, des acides aminés, des polyols notamment en C₂ à C₁₀, comme la glycérine, le sorbitol, le butylène glycol et le polyéthylène glycol, de l'urée, de l'allantoïne, des sucres et des dérivés de sucre, des vitamines hydrosolubles, de l'amidon, des extraits bactériens ou végétaux comme ceux d'*Aloe vera.*

Selon un autre mode de réalisation, dans les formes galéniques topiques, on peut utiliser plus particulièrement comme actifs additionnels lipophiles le rétinol (vitamine A) et ses dérivés, le tocophérol (vitamine E) et ses dérivés, les céramides, les huiles essentielles et les insaponifiables (tocotriénol, sésamine, gamma oryzanol, phytostérols, squalènes, cires, terpènes).

Comme actifs additionnels dans une formule galénique orale, on peut également considérer tous les ingrédients communément utilisés et/ou autorisés.

A titre illustratif, on peut citer les vitamines, les minéraux, les lipides essentiels, les oligoéléments, les polyphénols, les flavonoïdes, les phytoestrogènes, les antioxydants tels que l'acide lipoïque et la coenzyme Q10, les caroténoïdes, les prébiotiques, les protéines et les acides aminés, les mono et polysaccharides, les amino-sucres, les phytostérols et alcools triterpéniques d'origine végétale.

Il peut s'agir en particulier des vitamines A, C, D, E, PP et du groupe B, notamment B5, B6 et B8.

Parmi les caroténoïdes, on choisit de préférence, le bêta-carotène, le lycopène, la lutéine, la zéazanthine et l'astaxanthine.

Les minéraux et oligo-éléments particulièrement mis en oeuvre sont le zinc, le calcium, le magnésium, le cuivre, le fer, l'iode, le manganèse, le sélénium, le chrome (III).

Parmi les polyphénols, on peut retenir en particulier les polyphénols de raisin, de thé, d'olive, de cacao, de café, de pomme, de myrtille, de sureau, de fraise, de canneberge, et d'oignon.

De préférence parmi les phytoestrogènes, on retient les isoflavones sous la forme libre ou glycosylée, telles que la génistéine, la daidzéine, la glycitéine ou encore les lignanes, en particulier ceux du lin et du schizandra chinensis.

De préférence parmi les acides aminés convenant à l'invention on peut choisir la taurine, la thréonine, la cystéine, le tryptophane ou la méthionine, ou les peptides et les protéines les contenant.

De préférence parmi les lipides convenant à l'invention on peut choisir les lipides appartenant au groupe des huiles contenant des acides gras mono et polyinsaturés, tels que l'acide oléique, l'acide linoléique, l'acide alpha-linolénique, l'acide gamma-linolénique, l'acide stéaridonique, les acides gras oméga-3 de poisson à longue chaîne tels que l'EPA et le DHA, les acides gras conjugués issus de végétaux ou d'animaux tels que les CLA (Conjugated Linoleic Acid).

Ainsi, une composition destinée à une administration par voie orale peut en outre comprendre au moins un actif nutritionnel choisi parmi le lycopène, la vitamine C, la vitamine E et des composés polyphénols.

Une composition par voie orale de l'invention peut également comprendre d'autres actifs nutritionnels choisis parmi :
- les actifs nutritionnels anti-âge, tels que les antioxydants alimentaires, les nutriments aux propriétés anti-radicalaires et les cofacteurs des enzymes endogènes antioxydants, les vitamines A, C, E, les caroténoïdes, les xantophyles, les isoflavones, certains minéraux tels que le zinc, le cuivre, le magnésium, le sélénium, l'acide lipoïque, la co-enzyme Q10, la superoxyde dismutase (SOD) ou encore la taurine. Parmi les actifs anti-âges, on peut notamment citer les fractions insaponifiables extraits de lipides d'origine végétale, *Aloe vera,* le collagène marin natif ou hydrolysé, les huiles végétales ou marines riches en acides gras oméga-3, en oméga-6 (y compris l'acide gamma-linolénique),
- les actifs nutritionnels photoprotecteurs, tels que les antioxydants et les antiradicalaires, les vitamines A, C, E, les caroténoïdes, les xantophyles, certains minéraux tels que le zinc, le cuivre, le magnésium, le sélénium, la co-enzyme Q10, la superoxyde dismutase (SOD),
- les ingrédients nutritionnels présentant des propriétés d'hydratation ou encore immunomodulatrices tels que l'extrait de *Polypodium leucotomos,* les huiles végétales ou marines riches en acides gras oméga-3, en oméga-6, y compris l'acide gamma-linolénique.

Selon mode de réalisation, l'invention concerne un procédé cosmétique de prévention et/ou de traitement d'une altération du microrelief de la peau chez un individu en ayant besoin comprenant au moins une étape d'administration chez ledit individu d'au moins un microorganisme probiotique, notamment du genre *Lactobacillus sp..*

Selon un mode de réalisation, un procédé convenant à l'invention peut comprendre une étape consistant à observer une réduction de l'altération du microrelief, voire une amélioration du microrelief cutané.

Un procédé selon l'invention peut être mis en oeuvre, notamment, par voie topique par administration d'une composition cosmétique et/ou dermatologique, topique, telle que définie ci-dessus.

Avantageusement, un procédé de l'invention comprend l'application par voie topique, peut comprendre l'application d'une composition comprenant au moins un microorganisme probiotique conforme à l'invention, par exemple sous forme de masque, sur la peau.

Une telle administration peut être effectuée selon les techniques d'utilisation habituelles de ces compositions. Par exemple, elle peut consister en l'application de crèmes, de gels, de sérums, de lotions sur la peau ou les muqueuses.

Un procédé cosmétique topique selon l'invention peut être mis en oeuvre de façon journalière par exemple, à raison par exemple d'une une administration unique par jour ou d'une administration deux fois par jour, par exemple une fois le matin et une fois le soir.

Un procédé cosmétique topique selon l'invention peut être mis en oeuvre sur une période de temps variant d'une semaine à plusieurs semaines, voire plusieurs mois, cette période pouvant par ailleurs être répétée après des périodes de non traitement, pendant plusieurs mois voire plusieurs années.

A titre d'exemple l'administration par voie topique d'un microorganisme probiotique selon l'invention peut être répétée, par exemple, 2 à 3 fois par jour, ou plus, et généralement sur une durée prolongée d'au moins 4 semaines, voire 4 à 15 semaines, avec le cas échéant une ou plusieurs périodes d'interruption.

Un procédé cosmétique selon l'invention peut être mis en oeuvre par voie orale, notamment, par administration d'une composition alimentaire telle que définie ci-dessus.

Une quantité efficace de microorganisme peut être administré en une dose unique par jour ou en doses fractionnées sur la journée, par exemple deux à trois fois par jour.

Un procédé cosmétique orale peut être mis en oeuvre sur une période de temps variant d'une semaine à plusieurs semaines, voire plusieurs mois, cette période pouvant par ailleurs être répétée après des périodes de non traitement, pendant plusieurs mois voire plusieurs années.

A titre d'exemple l'administration par voie orale d'un microorganisme probiotique selon l'invention peut être effectuée à raison de, par exemple, 3 fois par jour plus, généralement sur une durée prolongée d'au moins 4 semaines, voire 4 à 15 semaines, comprenant ou non une ou plusieurs périodes d'interruption ou étant répétée après une période d'interruption.

### FIGURES

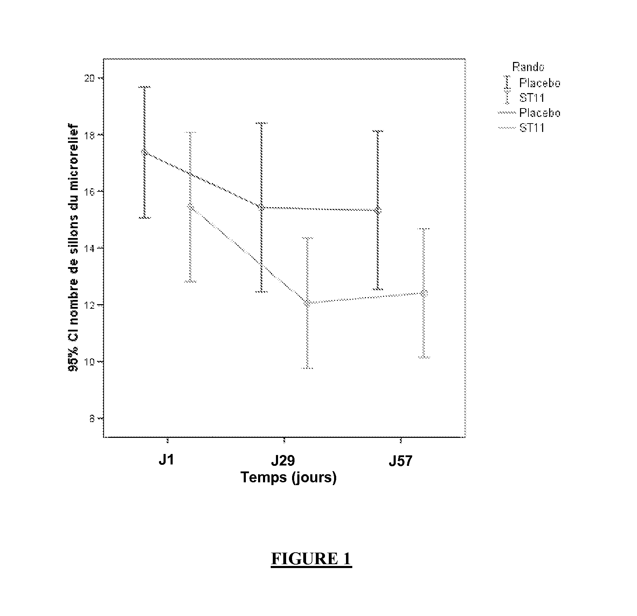

**Figure 1** : Illustre l'évolution du nombre de sillons du microrelief au niveau du coin de l'oeil au cours du temps, à J1, J29 et J57 chez les individus du groupe ST11 (ligne et symboles gris clair) et chez les individus du groupe placebo (ligne et symboles gris foncés). Le nombre de sillons du microrelief de la patte d'oie est nettement diminué chez les individus du groupe ST11. Les résultats sont exprimés en moyenne +/- intervalle de confiance.

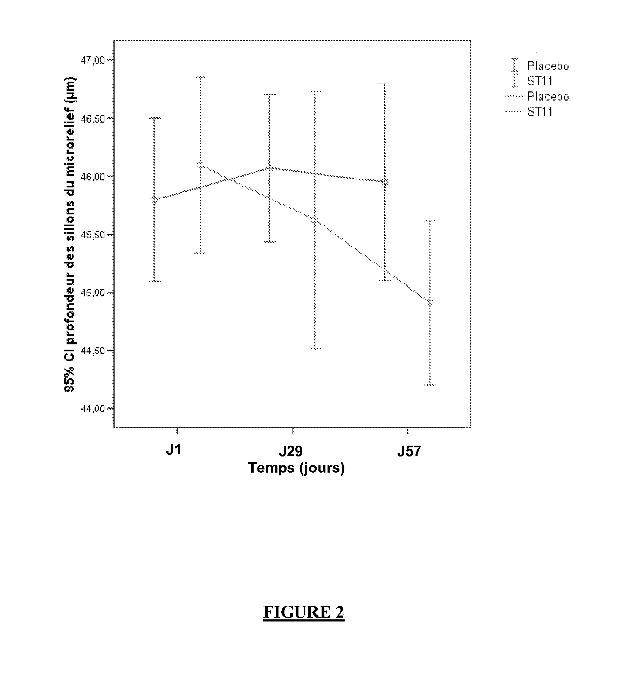

**Figure 2** : Illustre l'évolution de la profondeur des sillons du microrelief au niveau du coin de l'oeil au cours du temps, à J1, J29 et J57 chez les individus du groupe ST11 (ligne et symboles gris clair) et chez les individus du groupe placebo (ligne et symboles gris foncés). La profondeur des sillons du microrelief de la patte d'oie est nettement réduite chez les individus du groupe ST11. Les résultats sont exprimés en moyenne +/- intervalle de confiance.

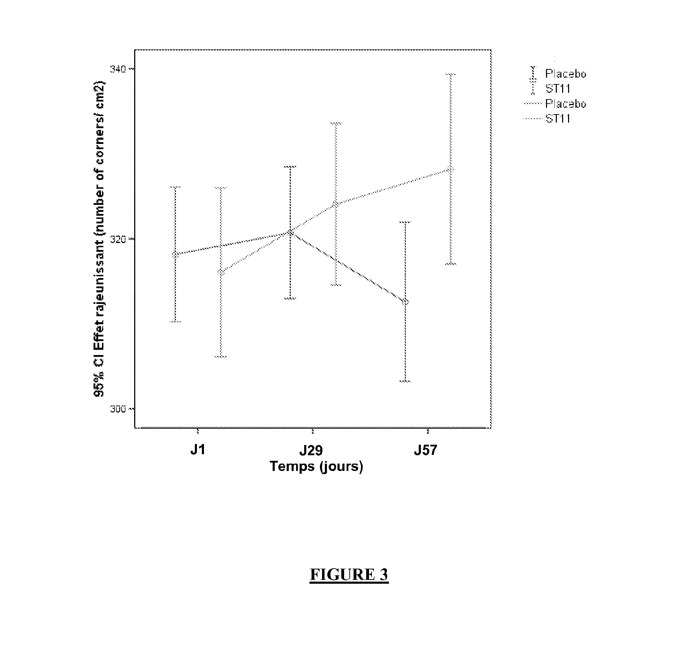

**Figure 3** : Evolution du nombre de corners au cours du temps, à J1, J29 et J57 chez les individus du groupe ST11 (ligne et symboles gris clair) et chez les individus du groupe placebo (ligne et symboles gris foncés). Le nombre de corners tend nettement à augmenter chez les individus du groupe ST11. Les résultats sont exprimés en moyenne +/intervalle de confiance.

Dans la description et dans les exemples suivants, sauf indication contraire, les pourcentages sont des pourcentages en poids et les plages de valeurs libellées sous la forme « entre ... et ... » incluent les bornes inférieure et supérieure précisées.

Les ingrédients sont mélangés, avant leur mise en forme, dans l'ordre et dans des conditions facilement déterminées par l'homme de l'art.

La teneur et la nature des ingrédients mis en oeuvre dans les compositions de l'invention sont ajustées par l'homme de l'art de sorte à ne pas affecter substantiellement les propriétés requises pour les compositions de l'invention.

Les exemples ci-après sont présentés à titre illustratif et non limitatif de l'invention.

### EXEMPLES

### Exemple 1

### Effet d'un complément alimentaire comprenant du Lactobacillus paracasei ST11 le microrelief cutané

### Matériels et méthodes

L'effet d'un complément alimentaire comprenant du *Lactobacillus paracasei* ST11 sur le microrelief cutané a été déterminé par réalisation d'une étude monocentrique, comparative, contrôlée, randomisée, et en double aveugle, sur 2 groupes parallèles de 33 sujets : complément alimentaire (groupe ST11) *vs* placebo (groupe placebo).

Les mesures sur le microrelief cutané ont été effectuées par utilisation d'un SKIN CHIP^{®} et par échographie.

Les sujets sélectionnés pour l'étude sont des individus masculins, sains, de plus de 60 ans, présentant au début de l'étude (J0) un grade de 4 à 5 au Densiscore^{®}, et, *a priori,* faiblement consommateurs de produits fermentés (moins de 125g/j).

Pendant l'étude, les volontaires ont accepté de ne pas consommer de produits fermentés contenant des bactéries vivantes (yaourts, fromages blancs, laits fermentés, fromages non pasteurisés, ...).

Le complément alimentaire a été formulé sous forme d'une poudre comprenant du *Lactobacillus paracasei* NCC 2461 (ST11) vivant à la dose totale de 10⁹ufc et de la maltodextrine. La poudre est à disperser ou dissoudre dans un liquide buvable, de préférence de l'eau.

Le placebo a été formulé sous forme d'une poudre comprenant uniquement de la maltodextrine. La poudre est à disperser ou dissoudre dans un liquide buvable, de préférence de l'eau.

Le traitement a été administré une fois par jour, pendant 61 jours. Les mesures ont été effectuées J-42 (préinclusion), J0, J1, J2, J3, J4, J5, J29, J30, J31, J32, J33, J57, J58, J59, J60 et J61.

Une analyse du microrelief cutané a été effectuée par réalisation d'empreintes au niveau du coin de l'oeil en polymère siliconé (SILFLO^{®}) au début de l'étude et à chaque temps de mesure, et mesure au Skin Image Analyser^{®} (SIA^{®}) selon le protocole suivant.

La surface de la réplique a été éclairée en lumière rasante à 35° pour générer des ombres portées qui ont ensuite été observées à l'aide d'une caméra CCD, reliée à un système informatique. Le champ étudié a été de 1 cm². L'image numérique obtenue a été analysée en niveaux de gris pour obtenir les différents paramètres caractérisant le relief de la surface cutanée.

Les paramètres étudiés à l'aide du logiciel QUANTIRIDES^{®} - MONADERM ont été la surface ridée totale, le nombre et la profondeur moyenne des sillons du microrelief cutané.

La profondeur maximale des sillons du microrelief a été fixée à 55 µm. La surface minimale d'un sillon pour qu'il soit pris en compte a été fixée arbitrairement à 0,03 mm².

Une évaluation du microrelief de la peau sur l'avant-bras gauche a également été effectuée à l'aide d'un SKIN CHIP^{®}.

Le SKIN CHIP^{®} est constitué d'une matrice de capteurs ST Microelectronics (256*360 à 500 dpi) mesurant la permittivité électrique. Cette propriété dépend de la distance peau/capteur et des propriétés de conduction de la peau donc, notamment, de son hydratation. Chaque capteur de la matrice donne une information codée sur 8 bits (0 à 255) qui, convertie en niveau de gris, constitue une image, permettant de visualiser la texture et le microrelief de la peau (Pixels noirs : surface de la sonde en contact avec la peau; Pixels blancs : sonde éloignée de la peau (fond d'un sillon du microrelief)).

### Résultats

### Nombre de sillons du microrelief cutané du visage (Figure 1)

Des empreintes en polymère siliconé (SILFLO®) ont été réalisées au niveau du coin de l'oeil en début d'étude et à chaque temps de mesure, puis étudiées au Skin Image Analyser^{®} (SIA^{®}). La profondeur maximale des sillons du microrelief a été fixée à 55 µm.

Les mesures obtenues pour les groupes ST11 et placebo apparaissent comparables à J1 (p=0,2585).

Seuls les volontaires du groupe ST11 ont observé une diminution significative du nombre de sillons du microrelief au niveau du coin de l'oeil au cours du temps (dès J29). A l'issue de la supplémentation, une diminution du nombre de sillons du microrelief de la patte d'oie de 19,6 % est observé pour le groupe ST11 et de 11,8 % pour le groupe placebo.

L'évolution entre J1 et J57 n'a pas paraît pas être différente entre les deux traitements (p=0,5181), bien qu'une tendance marquée en faveur du groupe ST11 puisse être observée.

**Tableau 1**

| Test de comparaison (p-values) intragroupe et intergroupe de l'évolution du nombre des sillons du microrelief au niveau du coin de l'oeil par rapport à J1 à tous les temps | | | |
|---|---|---|---|
| **Temps** | **PLACEBO (p value)** | **ST11 (p value)** | **PLACEBO/ST11 (p value)** |
| **J1-J29** | 0,3132 | 0,0060 | 0,4022 |
| **J1-J57** | 0,2080 | 0,0137 | 0,5181 |

### Profondeur des sillons du microrelief du visage (Figure 2)

Les mesures ont été effectuées sur les empreintes au niveau du coin de l'oeil comme décrit précédemment.

Les mesures obtenues entre le groupe ST11 et le groupe placebo apparaissent comparables à J1 (p=0,5533).

Seuls les volontaires du groupe ST11 voient la profondeur des sillons du microrelief au niveau du coin de l'oeil diminuer significativement entre J1 et J57. A l'issue de la supplémentation on observe une diminution de la profondeur des sillons du microrelief de 2,6 % pour les sujets du groupe ST11 et de 0,3 % pour les sujets du groupe placebo.

L'évolution entre J1 et J57 entre les deux groupes est différente en tendance (p=0,0556).

**Tableau 2 Tests de comparaison (p-values) intragroupe et intergroupe de l'évolution de la profondeur des sillons du microrelief au niveau du coin de l'oeil par rapport à J1 à tous les temps**

| **Temps** | **PLACEBO (p value)** | **ST11 (p value)** | **PLACEBO/ST11 (p value)** |
|---|---|---|---|
| **J1-J29** | 0,5897 | 0,3781 | 0,3082 |
| **J1-J57** | 0,7554 | 0,0185 | 0,0556 |

### Analyse du microrelief sur l'avant-bras à l'aide d'un SKIN-CHIP^{®} (Figure 3)

Le microrelief global est évalué par mesure du paramètre de densité de corner ou nombre de corners/cm². Ce paramètre représente le nombre de croisement des lignes fines primaires entre elles par cm² et est indicatif de l'orientation des lignes fines du microrelief. Une valeur élevée de ce paramètre est caractéristique d'une peau d'aspect jeune, lisse et en bonne santé.

Les mesures obtenues entre le groupe ST11 et le groupe placebo apparaissent comparables à J1 (p=0,7316).

Le nombre de corners/cm², dont l'augmentation traduit un effet rajeunissant, augmente significativement au cours du temps uniquement pour les individus du groupe ST11, alors que chez les individus du groupe placebo aucune variation significative n'est observée.

L'évolution entre J1 et J57 entre les deux groupes est significativement différente (p=0,0117) en faveur du groupe ST11. Ceci traduit un effet rajeunissant pour les sujets du groupe ST11 statistiquement significatif par rapport au groupe placebo.

A l'issue de la supplémentation avec un complément alimentaire selon l'invention, on observe une augmentation du nombre de corners de 3,8 % pour les sujets du groupe ST11, et de seulement 1,8 %. Pour le groupe placebo.

**Tableau 3 Tests de comparaison (p-values) intragroupe et intergroupe de l'évolution du nombre de corners/cm² par rapport à J1 à tous les temps**

| **Temps** | **PLACEBO (p value)** | **ST11 (p value)** | **PLACEBO/ST11 (p value)** |
|---|---|---|---|
| **J1-J29** | 0,5925 | 0,0543 | 0,2340 |
| **J1-J57** | 0,1324 | 0,0378 | 0,0117 |

### Conclusion

Les résultats obtenus dans l'étude clinique détaillée ci-dessus ont montré que l'administration orale d'un complément alimentaire comprenant du *Lactobacillus paracasei* (ST11) à la dose de 10⁹ ufc/j pendant deux mois permet d'obtenir :
- à la mesure du microrelief cutané par SIA^{®}
- une diminution du nombre de sillons du microrelief du visage et de leur profondeur au cours du temps uniquement pour le groupe ST11. La différence entre les deux traitements est significative en tendance (p=0,0556) en faveur du groupe ST11 lorsqu'on analyse l'évolution de la profondeur des sillons du microrelief entre J1 et J57.
- à la mesure du microrelief cutané par SKIN-CHIP^{®}
- une amélioration du réseau du microrelief uniquement chez les individus du groupe ST11 (J1/J57, p=0,0378). La différence est statistiquement significative en faveur du groupe lorsqu'on analyse l'évolution entre J1 et J57 (p=0,0117) ST11entre les deux traitements.

### Exemple 2

### Compositions topiques selon l'invention

### Exemple 2A

| | |
|---|---|
| *Lotion pour le visage* | |
| | % en poids |
| Poudre lyophilisée de *Lactobacillus paracasei* ST11 | 5,00 |
| Poudre lyophilisée de *Lactobacillusjohnsonii* | 5,00 |
| Anti-inflammatoire | 0,05 |
| Antioxydant | 0,05 |
| Isopropanol | 40,0 |
| Conservateur | 0,30 |
| Eau | qsp 100 |

### Exemple 2B

| | |
|---|---|
| *Gel pour le soin du visage* | |
| | % en poids |
| Poudre de *Lactobacillus paracasei* ST11 | 5,00 |
| Hydroxypropylcellulose (Klucel H^{®} vendu par la société HERCULES) | 5,00 |
| Vitamine E | 1,00 |
| Antioxydant | 0,05 |
| Isopropanol | 40,00 |
| Conservateur | 0,30 |
| Eau | qsp 100 |

### Exemple 2C

| | |
|---|---|
| *Lait pour le soin du visage* | |
| | % en poids |
| Poudre lyophilisée de *Lactobacillus paracasei* ST11 | 5,00 |
| Stéarate de glycérol | 1,00 |
| Alcool cétylstéarylique/alcool cétylstéarylique oxyéthyléné | |
| à 30 moles OE (Sinnowax AO^{®} vendu par la société HENKEL) | 3,00 |
| Alcool cétylique | 1,00 |
| Diméthicone (DC 200 Fluid^{®} vendu par la société DOW CORNING) | 1,00 |
| Huile de vaseline | 6,00 |
| Myristate d'isopropyle (Estol IMP 1514 vendu^{®} par UNICHEMA) | 3,00 |
| Glycérine | 20,00 |
| Conservateur | 0,30 |
| Eau | qsp 100 |

### Exemple 2D

| | |
|---|---|
| *Crème pour le soin du visage* | |
| | % en poids |
| Arachidyl béhenyl alcool/arachidylglucoside | 3,00 |
| Isohexadecane | 7,00 |
| Poudre de *Lactobacillus paracaseï* ST11 | 5,00 |
| Glycérine | 2,00 |
| Extrait de *Vitreoscilla filiformis* | 3,00 |
| BHT | 0,05 |
| POB méthyle | 0,10 |
| POB probyle | 0,05 |
| Eau | qsp 100 |

### Exemple 2E

| | |
|---|---|
| *Gel pour le soin du visage* | |
| | % en poids |
| Poudre de *Lactobacillus paracaseï* ST11 | 5,00 |
| Extrait de *Vitreoscilla filiformis* | 3,00 |
| Antioxydant | 0,05 |
| Vitamine E | 2,50 |
| Isopropanol | 40,00 |
| Conservateur | 0,30 |
| Eau | qsp 100 |

### Exemple 3

### Compositions pour la voie orale

### Exemple 3A

### Stick poudre

| Principe actif | |
|---|---|
| *Lactobacillus paracasei* ST11 | 10¹⁰ ufc |

| Excipient | |
|---|---|
| Gomme de xanthane | 0,8 mg |
| Benzoate de sodium | 0,2 mg |
| Maltodextrine | qsp 30 g |

On peut prendre un stick par jour.

### Exemple 3B

| | |
|---|---|
| *Capsule* | |
| | mg/capsule |
| *Lactobacillus johnsonii* | 10⁸ ufc |
| Vitamine C | 60,00 |
| Stéarate de magnésium | 0,02 |

On peut prendre une à trois de ces capsules par jour.

### Exemple 3C

| | |
|---|---|
| *Dragée* | |
| | mg/dragée |
| Matières actives | |
| *Lactobacillus paracaseï* ST11 | 5.10⁸ ufc |
| Excipient du noyau de la dragée | |
| Cellulose micro-cristalline | 70,0 |
| EncompressTM | 60,0 |
| Stéarate de magnésium | 3,0 |
| Silice colloïdale anhydre | 1,0 |
| Agent d'enrobage | |
| Gomme laque | 5,0 |
| Talc | 61,0 |
| Saccharose | 250,0 |
| Polyvidone | 6,0 |
| Dioxyde de titane | 0,3 |
| Agent de coloration | 5,0 |

Ce type de dragée peut être pris 1 à 3 fois par jour.

### Exemple 3D

| | |
|---|---|
| *Dragée* | |
| | mg/dragée |

| Matières actives | |
|---|---|
| *Lactobacillus paracaseï* ST11 | 10⁹ ufc |
| *Lactobacillus johnsonii* | 10⁹ ufc |

| Excipient du noyau de la dragée | |
|---|---|
| Cellulose micro-cristalline | 70,0 |
| EncompressTM | 60,0 |
| Stéarate de magnésium | 3,0 |
| Silice colloïdale anhydre | 1,0 |

| Agent d'enrobage | |
|---|---|
| Gomme laque | 5,0 |
| Talc | 61,0 |
| Saccharose | 250,0 |
| Polyvidone | 6,0 |
| Dioxyde de titane | 0,3 |
| Agent de coloration | 5,0 |

Ce type de dragée peut être pris 1 à 3 fois par jour.

## Revendications

1. Utilisation cosmétique d'au moins un microorganisme probiotique, notamment du genre *Lactobacillus sp.,* à titre d'actif utile pour traiter et/ou prévenir une altération du microrelief de la peau, ledit microorganisme probiotique étant distinct d'un *Lactobacillus johnsonii* formulé dans une composition comprenant en poids:
- 875,42 g de lait entier 1,7 %,
- 10 g de lait écrémé 1,0 %,
- 30 g de yaourt Nestlé LC1,
- 70 g de sucre,
- 3 g de stabilisant,
- 0,08 de saveur tangerine,
- 0,93 g de teavigo,
- 0,17 g de génistéine TG,
- 0,14 g de resvératrol,
- 0,24 g de β-carotène 7 % CWS,
- 10 g d'huile alimentaire ROPUFA '30' n-3.

2. Utilisation selon la revendication précédente, pour réduire le nombre de sillons du microrelief de la peau.

3. Utilisation selon les revendications 1 ou 2, pour réduire la profondeur des sillons du microrelief de la peau.

4. Utilisation selon l'une quelconque des revendications précédentes, dans laquelle le microrelief est le microrelief de la peau du visage.

5. Utilisation selon l'une quelconque des revendications précédentes, dans laquelle ledit microorganisme probiotique est administré par voie orale, topique ou parentérale, et en particulier par voie orale.

6. Utilisation selon l'une quelconque des revendications précédentes, dans laquelle ledit microorganisme probiotique est mis en oeuvre sous une forme vivante, semi-activée, inactivée ou morte.

7. Utilisation selon l'une quelconque des revendications précédentes, dans laquelle ledit microorganisme probiotique est mis en oeuvre sous forme d'un lysat.

8. Utilisation selon l'une quelconque des revendications précédentes, dans laquelle ledit microorganisme probiotique est choisi parmi des *Lactobacillus sp.,* des *Bifidobacterium sp.,* des *Cocci,* des levures, des bactéries sporulées, et leurs mélanges.

9. Utilisation selon l'une quelconque des revendications précédentes, dans laquelle ledit microorganisme probiotique est un *Lactobacillus paracasei,* et en particulier *Lactobacillus paracasei* CNCM I-2116 (ST11).

10. Utilisation selon l'une quelconque des revendications précédentes, dans laquelle ledit microorganisme probiotique est mis en oeuvre dans une composition à raison de 0,0001 à 30 %, en particulier à raison de 0,001 à 20 % et plus particulièrement à raison de 0,01 à 15 % en poids, en particulier de 0,1 à 10 % en poids, et notamment de 1 % à 5 % en poids par rapport au poids total de la composition le contenant.

11. Utilisation selon l'une quelconque des revendications 1 à 9, dans laquelle ledit microorganisme probiotique est mis en oeuvre vivant par voie orale à raison de de 10³ à 10¹⁵ ufc/g, en particulier de 10⁵ à 10¹⁵ ufc/g, et plus particulièrement de 10⁷ à 10¹² ufc/g de microorganismes vivants par gramme de composition le contenant.

12. Utilisation selon l'une quelconque des revendications précédentes, dans laquelle ledit microorganisme probiotique est mis en oeuvre en association avec un actif additionnel.

13. Procédé cosmétique de prévention et/ou de traitement de l'altération du microrelief de la peau comprenant l'administration d'au moins un microorganisme probiotique, notamment du genre *Lactobacillus sp.,* tel que défini selon l'une quelconque des revendications 5 à 11, ledit microorganisme probiotique étant distinct d'un *Lactobacillus johnsonii* formulé dans une composition comprenant en poids:
- 875,42 g de lait entier 1,7 %,
- 10 g de lait écrémé 1,0 %,
- 3 0 g de yaourt Nestlé LC1,
- 70 g de sucre,
- 3 g de stabilisant,
- 0,08 de saveur tangerine,
- 0,93 g de teavigo,
- 0,17 g de génistéine TG,
- 0,14 g de resvératrol,
- 0,24 g de β-carotène 7 % CWS,
- 10 g d'huile alimentaire ROPUFA '30' n-3.
